# EUROPEAN PATENT APPLICATION

(11) **EP 1 897 572 A1**
(43) Date of publication of application: **12.03.2008**
(21) Application number: 07425556.3
(22) Date of filing: 10.09.2007
(51) Int. Cl.: A61M 3/02

(54) **Container for forced irrigation, particularly for ocular surgery intervention**

(30) Priority: 11.09.2006 IT RM20060477
(71) Applicant: Optikon 2000 S.p.a., 00138 Roma (IT)
(72) Inventor: Angelini, Giampiero, 00138 Roma (IT); Longo, Dario, 00138 Roma (IT); Di Florio, Armando, 00138 Roma (IT)
(74) Representative: Iannone, Carlo Luigi

(57) **Abstract**

The invention relates to a container (1) for forced irrigation, particularly for ocular surgery intervention, comprised of three elements (2, 3, 4), provided side by side, coupled each other along their perimetral edge, so as to realise a first (5) and a second (6) pocket, said pockets (5, 6) being provided with independent substance introduction and withdrawal means (8, 9), contents of the two pockets (5, 6) not being physically in communication each other.

## Description

The present invention relates to a container for forced irrigation, particularly for ocular surgery intervention.

More specifically, the invention concerns a container for forced irrigation, particularly for ocular surgery intervention, permitting obtaining a high degree of sterility of sterile saline solution, necessary to irrigate ocular bulb during surgical interventions.

As it is well known, liquids are sucked during ocular surgical interventions. This requires need of replacing said liquids, in order to prevent emptying of ocular bulb. It is usually carried out by introduction of sterile saline solution into the eye.

Injection of the above liquid must be carried out with a pressure depending on surgical technique used. It is required balancing flow of sucked liquids, varying and not fixed, without too large differences of eye inner pressure with respect to the standard values. However, sometimes, it is necessary that injection pressure of saline solution is higher than ocular pressure, for example in order to suddenly stop haemorrhages. To solve these unforeseeable situations, occurring during a surgical intervention, it would be preferable having a system that can vary pressure of saline solution, ideally immediately.

In a known system for adjusting pressure of solution injected within the eye during a surgical intervention, a bottle is used containing the sterile saline solution, hanging above the patient head. Irrigation pressure is set by adjustment of bottle height.

In fact, irrigation pressure is directly proportional to the difference of height between the bottle and the patient eye, taking into consideration that a difference of one meter corresponds to a pressure of 76 mmHg.

By the simple method described in the above, irrigation pressure can be modified by the variation of the height of the bottle with respect to the patient.

It is evident that this operation is rather slow and, as already said, quickness is very important during the intervention.

In order to solve these problems, different systems have been realised in order to pressurise irrigation liquid, for example by controlled pumping of air within the bottle containing the saline solution.

Air controlled pumping systems, although partially solving the problem of quickness of response of saline solution injection pressure variation, have a different problem. In fact, air pumped for compressing the solution comes in physical contact with the same solution, thus being it necessary sterilising the same. To this end, suitable filters are usually provided downward the pump, with a filtering mesh of 0.22 micron or less. This kind of filters slower the airflow both when increasing pressure and when decreasing pressure. This involves a slowing down of response speed of the system for varying endo-ocular pressure.

It is well evident that this procedure is not particularly efficient with respect to the response quickness. Furthermore, system requires additional costs for sterilisation filter. Under dynamic conditions, since pressure of introduced air is usually controlled and adjusted upward the sterilisation filter, a measurement error is present due to impendence of the same filter; effective pressure is always lower that the set one, unless more complex systems are provided having an additional flow sensor. In view of the above, it is well evident the needing of having a system ensuring quickness in case of pressure variation and not requiring filtering operations, that, besides complicating the whole system, increases its costs, as the one suggested according to the present invention, permitting completely eliminating the air filtering system.

In view of the above, the same Applicant has realised and suggested a solution, described in the Italian Patent n° 1,333,782 (filed on December 2, 2002), comprising a system permitting air pumping, with a quick response, not requiring an air filtering, and ensuring at the same time a high hygiene standard.

These results are obtained by the solution described and claimed in the above mentioned Italian patent n° 1,333, 782, by a container for forced irrigation, that can be particularly used for ocular surgery interventions, comprising a first and a second pockets, such that the first pocket completely, or partially, containing the second pocket, and further characterised in that contents of the two pockets do not communicate each other.

Solution suggested by the above Italian patent, even permitting obtaining the above technical results, has two practical problems that are overcome by the solution according to the present invention.

A problem of the known solution providing the two pockets one inside the other one is that surgeon and other personnel can observe only the outer pocket, which appears always inflated (being pressurised). For this reason, it is practically impossible keeping under visual control amount of saline solution still available within the inner pocket. This causes a risk: liquid could suddenly exhaust during the surgical intervention causing collapse of the front eye chamber.

Main object of the present invention is therefore that of providing a solution permitting having a higher volume within the pockets.

Another object of the present invention is that of providing a solution that is very simple to be realised.

It is therefore specific object of the present invention a container for forced irrigation, particularly for ocular surgery intervention, comprised of three elements, provided side by side, coupled each other along their perimetral edge, so as to realise a first and a second pocket, said pockets being provided with independent substance introduction and withdrawal means, contents of the two pockets not being physically in communication each other.

Always according to the invention, said container is comprised of soft material.

Furthermore, according to the invention, at least a first tube is connected with said first pocket.

Still according to the invention, said soft material is a plastic material.

Preferably, according to the invention, said soft material is polyvinilchloride (PVC).

Furthermore, the invention can provide a hole for hanging the container to a support for forced irrigation.

Still according to the invention, a vertical line is printed on the central element, comprised of PVC, separating the saline solution with respect to air, said vertical line extending all along its length, with a horizontal line printed on the front element of the container.

At the beginning of the surgical intervention, when the pocket is full of saline solution, dividing septum is squeezed on the rear side of the pocket. While the pocket empties, septum is pushed toward the front wall of the pocket (when the pocket is almost empty, septum begins adhering to the front wall) and vertical line is always more clearly viewable.

A horizontal line is printed on the front wall of the container: when the vertical line on the dividing septum crosses with the horizontal line, about 100 cc of saline solution are available.

This permits to the personnel of visually verifying the amount of saline solution still available.

The present invention will be now described, for illustrative but not limitative purposes, according to its preferred embodiments, with particular reference to the figures of the enclosed drawings, wherein:

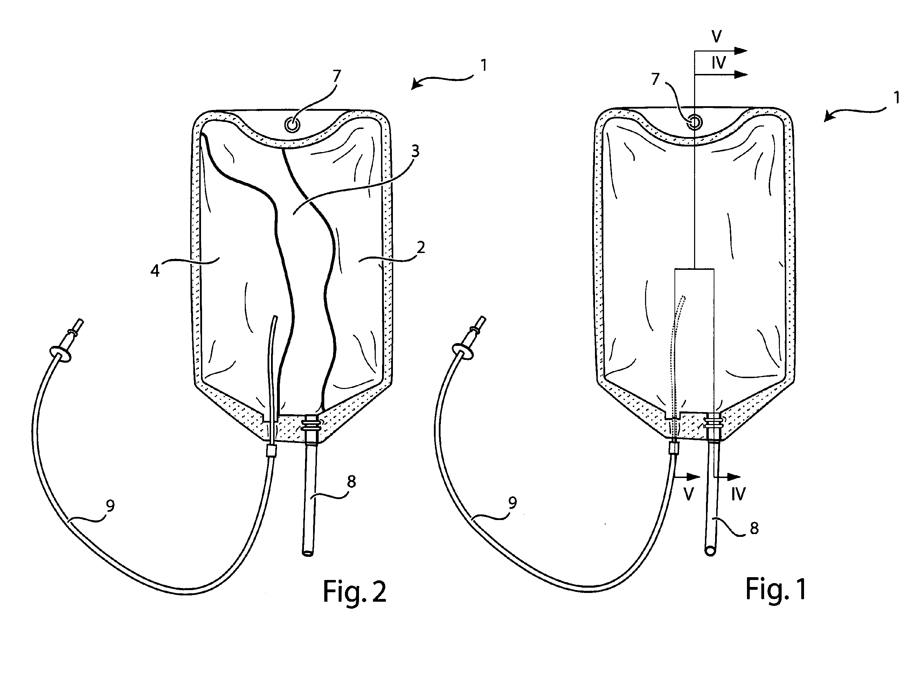

figure 1 is a schematic view of a container for forced irrigation according to the invention;
figure 2 shows the container of figure 1 partially broken away;

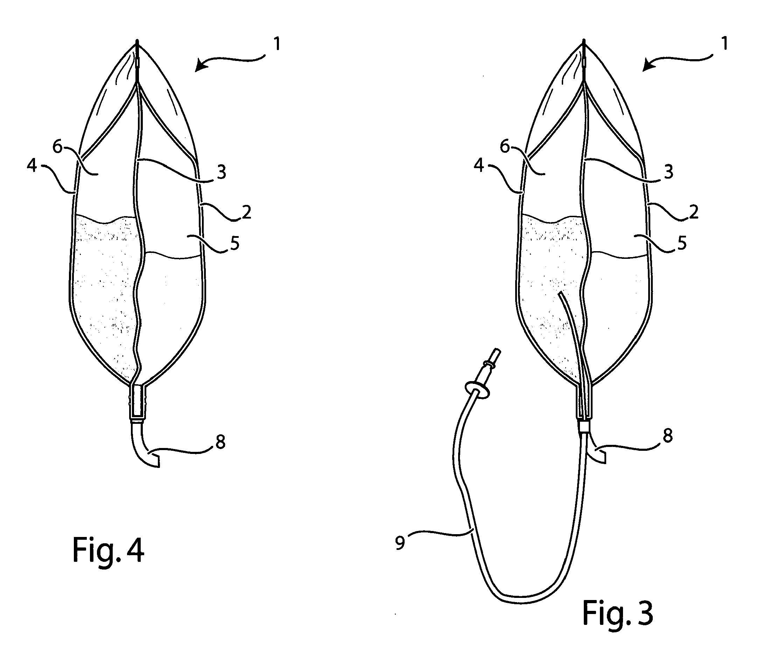

figure 3 is a section view taken along line III-III of figure 1; and
figure 4 is a section view taken along line IV-IV of figure 1.

Observing specifically figures of the enclosed drawings, it can be observed a contained according to the invention, generically indicated by reference number 1.

Container 1 is comprised by three side by side sheets, respectively indicated by reference numbers 2, 3 and 4, coupled each other along their perimetral edge, so as to realise two separated pockets 5, 6, not communicating each other.

Sheets 2, 3, 4 are comprised of soft and sterile material, for example in the specific case comprised of PVC.

Pocket 5 contains air, while pocket 6 contains the sterile saline solution. Two pockets 5, 6 are completely insulated each other, thus air contained within pocket 5 and sterile saline solution, contained within pocket 6, never are in contact each other.

During a surgical intervention, container 1 according to the invention can be hanged at a hook by the hole 7.

Pocket 6 is filled with sterile saline solution to be injected within the ocular bulb. Air is introduced within the pocket 5, with a controlled pressure, through the tube 8, according to direction A.

Air injected in this way within the pocket 5 does not require a sterilisation. Solution contained within pocket 6 does not come in contact with air. This implies possibility of eliminating air filters that, as already said, slower response times of the irrigation systems.

After the pumping of the air inside the pocket 5, it is obtained compression of the pocket 6, with the consequent forced injection of the saline solution, according to direction B, within the ocular bulb by tube 9. injection of solution is substantially at controlled pressure and absolutely sterilised without the use of further air filtering and sterilisation means.

Among the advantages obtained by the solution according to the present invention, it is possible ensuring a high hygiene degree, and at the same time with a high injection response speed of sterile solution. Another advantage concerns possibility of avoiding air filtering with a controlled pressure, making the whole system more economic, since complex flow sensors and pneumatic and electronic circuits are not necessary, and that were provided for optimisation of injection pressure.

The present invention has been described for illustrative but not limitative purposes, according to its preferred embodiments, but it is to be understood that modifications and/or changes can be introduced by those skilled in the art without departing from the relevant scope as defined in the enclosed claims.

## Claims

1. Container for forced irrigation, particularly for ocular surgery intervention, **characterised in that** it is comprised of three elements, provided side by side, coupled each other along their perimetral edge, so as to realise a first and a second pocket, said pockets being provided with independent substance introduction and withdrawal means, contents of the two pockets not being physically in communication each other.

2. Container for forced irrigation according to claim 1, **characterised in that** said container is comprised of soft material.

3. Container for forced irrigation according to one of the preceding claims, **characterised in that** at least a first tube is connected with said first pocket.

4. Container for forced irrigation according to one of the preceding claims, **characterised in that** at least a second tube is connected with said second pocket.

5. Container for forced irrigation according to one of the preceding claims, **characterised in that** said soft material is a plastic material.

6. Container for forced irrigation according to one of the preceding claims, **characterised in that** said soft material is polyvinilchloride (PVC).

7. Container for forced irrigation according to one of the preceding claims, **characterised in that** it is provided a hole for hanging the container to a support for forced irrigation.

8. Container for forced irrigation according to one of the preceding claims, **characterised in that** a vertical line is printed on the central element, comprised of PVC, separating the saline solution with respect to air, said vertical line extending all along its length, with a horizontal line printed on the front element of the container.

9. Container for forced irrigation according to each one of the preceding claims, substantially as illustrated and described.
